# FASCICULE DE BREVET EUROPEEN

(11) **EP 2 708 114 B1**
(45) Date de publication et mention de la délivrance du brevet: **06.04.2016**
(21) Numéro de dépôt: 13182550.7
(22) Date de dépôt: 02.09.2013
(51) Int. Cl.: A01G 7/00, A01G 9/24

(54) **Dispositif de régulation de la pression gazeuse d'une enceinte fermée soumise à des variations de température**
Gasdruck-Regulierungsvorrichtung eines geschlossenen Behälters, der Temperaturschwankungen ausgesetzt wird
Device for adjusting the gaseous pressure of a sealed enclosure subject to temperature variations

(30) Priorité: 14.09.2012 FR 1258632
(43) Date de publication de la demande: 19.03.2014
(73) Titulaire: JF Cesbron, 49480 Saint-Sylvain-d'Anjou (FR); Centre National de la Recherche Scientifique, 75016 Paris (FR)
(72) Inventeur: Verdier, Bruno, 77000 Melun (FR); Jouanneau, Isabelle, 77710 Treuzy le Velay (FR); Simonnet, Benoît, 44980 Sainte Luce sur Loire (FR); Cherbonnier, Thierry, 49800 Brain sur l'Authion (FR); Rabin, Christian, 72200 La Fleche (FR); Hallet, Yoann, 49250 Beaufort en Vallée (FR)
(74) Mandataire: Godineau, Valérie

(56) Documents cités:
- EP-A2- 2 180 038
- CN-B- 101 480 144
- DE-A1- 10 222 438
- FR-A1- 2 582 472

## Description

La présente invention concerne un dispositif et un procédé pour l'homogénéisation et la régulation notamment en teneur en gaz et en pression de l'atmosphère gazeuse d'une enceinte étanche, telle qu'une cellule climatique.

Des cellules d'étude environnementales, encore appelées cellules climatiques, du type de celles décrites dans le brevet FR 2.937.653, ou encore DE 102 22 488, sont équipées de moyens de génération d'un environnement climatique apte à modifier l'état de l'atmosphère dans ladite cellule, cet état se caractérisant par des paramètres tels que température, humidité, ensoleillement, pression, vent, précipitations, teneur en gaz...

Dans certaines conditions il peut être souhaitable de faire varier l'un des paramètres cités ci-dessus, à savoir la température, l'humidité, le vent, les précipitations sans faire varier la pression et la teneur en gaz à l'intérieur de ladite enceinte.

Jusqu'à présent, pour permettre l'obtention d'un tel résultat, l'enceinte est associée à un ballon à vessie qui peut être gonflé/dégonflé au gré des variations de pression. Toutefois, cette solution est limitée en termes de réactivité et de sensibilité à des variations de pression très faibles. En outre, une telle solution ne permet pas de couvrir une large plage de pressions sans amener à une installation de grand encombrement. Enfin, une telle solution ne permet pas de maintenir la plus constante possible la teneur en gaz de l'enceinte et oblige à une modification importante de la teneur en gaz de l'enceinte à chaque aspiration ou injection d'air dans ladite enceinte.

Un but de la présente invention est donc de proposer un dispositif du type précité dont la conception permet une réactivité plus importante en termes de variation de pression sans augmenter l'encombrement du dispositif.

Un autre but de la présente invention est de proposer un dispositif de type précité dont la conception permet de couvrir une large plage de variation de pression et de réguler la pression sans modifier de manière substantielle la teneur en gaz de l'enceinte.

A cet effet, l'invention a pour objet un dispositif pour l'homogénéisation et la régulation de l'atmosphère gazeuse d'une enceinte étanche, telle qu'une cellule climatique,
caractérisé en ce que le dispositif comprend une pluralité de réservoirs disposés en série, chaque réservoir étant relié à un réservoir voisin par un circuit de liaison équipé d'une pompe à air apte à aspirer l'air dans le réservoir, dit amont, de ladite paire de réservoirs voisins pour le rejeter dans le réservoir, dit aval, le réservoir le plus en amont de ladite série, dit premier réservoir, et le réservoir le plus en aval de la série, dit dernier réservoir, étant équipés respectivement l'un, d'au moins deux entrées d'air raccordables chacune par un circuit, dit d'aspiration d'air, obturable, à l'enceinte à réguler, et l'autre, d'au moins deux sorties d'air raccordables chacune par un circuit, dit d'injection d'air, obturable, à l'enceinte à réguler, les organes d'obturation des deux ou d'au moins deux des circuits d'aspiration étant du type à commande en ouverture/fermeture indépendante d'un organe d'obturation à un autre et les organes d'obturation des deux ou d'au moins deux des circuits d'injection étant du type à commande en ouverture/fermeture indépendante d'un organe d'obturation à un autre.

La présence d'au moins deux réservoirs permet de disposer d'un réservoir basse pression qui tend à aspirer l'air contenu dans l'enceinte à réguler lors de l'ouverture du circuit d'aspiration le reliant à ladite enceinte, et un réservoir haute pression qui tend à injecter de l'air dans l'enceinte lors de l'ouverture du circuit d'injection le reliant à ladite enceinte.

Il est donc possible de réguler, dans le sens d'une réduction de la pression à l'aide de l'un des réservoirs, et dans le sens d'une augmentation de la pression à l'aide de l'autre réservoir, la pression régnant à l'intérieur de l'enceinte.

Comme chaque réservoir d'extrémité de ladite série de réservoirs est raccordé à l'enceinte par l'intermédiaire d'au moins deux circuits, il est possible de maintenir de manière permanente ouvert l'un des circuits de manière à aspirer de manière permanente de l'air au niveau du réservoir basse pression, et à injecter de manière permanente de l'air au niveau du réservoir haute pression pour assurer une homogénéité entre l'air contenu dans l'enceinte à réguler et celui contenu dans les réservoirs.

Cette reprise d'air et ce débit de fuite peuvent être contrôlés de sorte que le débit d'air repris est égal au débit d'air injecté pour ne pas générer de variations de pression à l'intérieur de l'enceinte tout en assurant une régénération de l'air de l'enceinte et une homogénéité de l'air de l'enceinte et des réservoirs.

Lorsqu'un ventilateur est mis en marche à l'intérieur de l'enceinte pour générer du vent ou lorsque la température est augmentée pour matérialiser l'ensoleillement, la pression varie à l'intérieur de l'enceinte. Cette pression peut être maintenue constante à l'aide du deuxième circuit d'admission et/ou du deuxième circuit d'injection grâce au fait que les moyens de commande des organes d'obturation sont configurés pour permettre, d'une part, la commande en ouverture/fermeture de l'organe d'obturation de l'un des circuits d'admission indépendamment de la commande en ouverture/fermeture de l'organe d'obturation de l'autre ou d'un autre circuit d'admission, et d'autre part, la commande en ouverture/fermeture de l'organe d'obturation de l'un des circuits d'injection indépendamment de la commande en ouverture/fermeture de l'organe d'obturation de l'autre ou d'un autre circuit d'injection.

Une telle conception peut permettre, en parallèle de la fermeture de l'organe d'obturation de l'un des circuits d'aspiration, le maintien à l'état ouvert de l'organe d'obturation de l'autre ou d'un autre circuit d'aspiration. De la même manière, il est possible, en parallèle de la fermeture de l'organe d'obturation de l'un des circuits d'injection, d'obtenir le maintien à l'état ouvert de l'organe d'obturation de l'autre ou d'un autre circuit d'injection.

L'architecture retenue peut permettre également de multiplier les circuits d'aspiration et d'injection pour obtenir un réglage très fin et très réactif en variation de pression. Pour obtenir un réglage très fin et très réactif de la pression à l'intérieur de l'enceinte, l'un des circuits d'aspiration, ou respectivement d'injection, peut être dédié à une variation faible de la pression à l'intérieur de l'enceinte, un autre à une variation forte de la pression à l'intérieur de l'enceinte, le dernier au circuit de régénération par génération d'un flux d'air permanent entre réservoirs et enceinte.

Il en est de même lors de la multiplication des réservoirs qui permet de travailler sur des plages de pression très étendues.

En résumé, un tel dispositif permet une régularisation fine et rapide de la pression sur une large plage à l'intérieur de l'enceinte sans avoir à disposer de pompes à vide ou de réservoirs surdimensionnés et de conserver une homogénéité de la qualité de l'air de l'ensemble formé de l'enceinte et des réservoirs.

De préférence, le dispositif comporte au moins deux modes de fonctionnement, l'un, dit mode de chargement en air des réservoirs dans lequel la pompe à air du ou d'au moins l'un des circuits de liaison est raccordée en entrée à l'air extérieur, l'autre, dit mode de régulation, dans lequel la pompe à air du ou d'au moins l'un des circuits de liaison est raccordée en entrée au réservoir immédiatement en amont.

Ce mode de chargement en air des réservoirs est utilisé au moment du démarrage du dispositif pour amener les réservoirs d'air aux pressions souhaitées avec un air contenu dans les réservoirs identique à celui de l'enceinte.

De préférence, le réservoir le plus en amont de ladite série, dit premier réservoir, et le réservoir le plus en aval de la série, dit dernier réservoir, sont équipés respectivement l'un, d'au moins trois entrées d'air raccordables chacune par un circuit dit d'aspiration d'air obturable à l'enceinte à réguler et l'autre, d'au moins trois sorties d'air raccordables chacune par un circuit d'injection d'air obturable à l'enceinte à réguler.

Il en résulte un gain en réactivité du dispositif comme mentionné ci-dessus, le dispositif étant extrêmement réactif en cas de grandes variations de pression ou de faibles variations de pression à l'intérieur de l'enceinte.

De préférence, le ou au moins l'un des circuits de liaison entre deux réservoirs de la série comporte un organe d'obturation.

Ledit organe d'obturation du circuit de liaison est une vanne trois voies commutable entre au moins deux positions, et est apte à passer d'une position dans laquelle le réservoir immédiatement en aval dudit organe d'obturation est apte à être alimenté en air à partir de l'air contenu dans le réservoir immédiatement en amont, à une position dans laquelle le réservoir immédiatement en aval dudit organe d'obturation est apte à être alimenté en air à partir d'air extérieur auxdits réservoirs.

Il est ainsi aisé de passer du mode de chargement des réservoirs au mode de régulation par simple commutation de la ou des vanne(s).

De préférence, chaque réservoir est équipé d'au moins une sortie d'échappement d'air obturable débouchant à l'extérieur du dispositif, à l'air libre.

De préférence, chaque réservoir est équipé d'au moins deux sorties d'échappement d'air obturables débouchant à l'extérieur du dispositif, à l'air libre, l'organe d'obturation de l'une des sorties étant un organe d'obturation de type soupape sensible à la pression et apte à passer automatiquement de la position fermée à la position ouverte en fonction de la pression régnant à l'intérieur du réservoir, l'organe d'obturation de l'autre sortie d'échappement étant à actionnement manuel ou commandé.

La présence de deux organes d'obturation permet d'assurer une parfaite sécurité du dispositif en toute circonstance.

De préférence, chaque réservoir est équipé de moyens de mesure de la pression régnant à l'intérieur dudit réservoir et en ce que chaque pompe à air est équipée de moyens de pilotage de ladite pompe en fonction des données fournies par lesdits moyens de mesure de la pression.

De préférence, dans le cas d'un dispositif comprenant au moins trois réservoirs, lesdits moyens de pilotage sont configurés pour permettre le fonctionnement de la pompe à air disposée sur le circuit de liaison entre le premier réservoir et le réservoir immédiatement en aval tant que la pression à l'intérieur du premier réservoir est supérieure à une pression prédéterminée, et de la pompe à air disposée sur le circuit de liaison entre le dernier réservoir et le réservoir immédiatement en amont tant que la pression à l'intérieur du dernier réservoir est inférieure à une pression prédéterminée.

De préférence, le dispositif comporte des moyens de mesure de la pression à l'intérieur de l'enceinte à réguler et les moyens de commande en ouverture/fermeture des organes d'obturation des circuits d'aspiration et d'injection sont aptes à émettre des signaux de commande desdits organes d'obturation en fonction des données fournies par les moyens de mesure de la pression à l'intérieur de l'enceinte à réguler.

L'invention a encore pour objet un procédé d'homogénéisation et de régulation de l'atmosphère gazeuse d'une enceinte étanche, telle qu'une cellule climatique, à l'aide d'un dispositif conforme à celui décrit ci-dessus,
caractérisé en ce que les premier et dernier réservoirs étant respectivement, l'un, un réservoir basse pression, l'autre, un réservoir haute pression, ledit procédé comprend une étape de régénération de l'air de l'enceinte à réguler par ouverture de l'un des circuits d'aspiration et de l'un des circuits d'injection d'air et une étape de régulation de l'atmosphère gazeuse de l'enceinte par ouverture d'un circuit d'aspiration autre que celui sollicité lors de l'étape de régénération et par ouverture d'un circuit d'injection autre que celui sollicité lors de l'étape de régénération en fonction de la pression régnant à l'intérieur de ladite enceinte à réguler.

L'invention a encore pour objet une installation de génération d'un environnement climatique pour l'étude de son impact sur au moins un échantillon incluant au moins un être vivant, ladite installation comportant au moins une cellule climatique d'étude environnementale apte à recevoir un échantillon à étudier, des moyens de génération d'un environnement climatique apte à modifier l'état de l'atmosphère de la ou de chaque cellule climatique, un système électronique et/ou informatique de commande de l'installation, ledit système pilotant au moins les moyens de génération d'un environnement climatique, et un dispositif d'homogénéisation et de régulation de l'atmosphère gazeuse d'au moins une cellule climatique, caractérisée en ce qu'elle comporte un dispositif du type précité.

L'invention sera bien comprise à la lecture de la description suivante d'exemples de réalisation, en référence aux dessins annexés dans lesquels :
La figure 1 représente une vue schématique d'un dispositif à deux réservoirs ;
La figure 2 représente une vue schématique d'un dispositif à trois réservoirs. Comme mentionné ci-dessus, le dispositif pour l'homogénéisation notamment en teneur en gaz, et la régulation en pression de l'atmosphère gazeuse d'une enceinte étanche est plus particulièrement destiné à être appliqué à une cellule 40 climatique même si d'autres applications peuvent être envisagées sans sortir du cadre de l'invention.

Comme mentionné ci-dessus, le dispositif comprend une pluralité de réservoirs disposés en série. Chaque réservoir est relié à un réservoir voisin par un circuit de liaison équipé d'une pompe à air. Ladite pompe à air est apte à aspirer l'air dans le réservoir, dit amont, de ladite paire de réservoirs voisins pour le rejeter dans le réservoir, dit aval, de la paire de réservoirs voisins.

Le réservoir 1 le plus en amont de la série, appelé premier réservoir, et le réservoir le plus en aval de la série, appelé dernier réservoir 2, sont équipés respectivement l'un de trois entrées 7, 8, 9 d'air raccordables chacune par un circuit 13, 14, 15 dit d'aspiration obturable à l'enceinte à réguler et l'autre de trois sorties 10, 11, 12 d'air raccordables chacune par un circuit 16, 17, 18 dit d'injection obturable à l'enceinte 40 à réguler.

Les organes 19, 20, 21 d'obturation d'au moins deux des trois circuits 13, 14, 15 d'aspiration sont du type à commande en ouverture/fermeture indépendante d'un organe d'obturation à un autre.

Les organes 22, 23, 24 d'obturation d'au moins deux des circuits 16, 17, 18 d'injection sont du type à commande en ouverture/fermeture indépendante d'un organe d'obturation à un autre.

Dans les exemples représentés, tous les organes d'obturation des circuits d'injection et des circuits d'aspiration sont à commande en ouverture/fermeture indépendante d'un organe d'obturation à un autre.

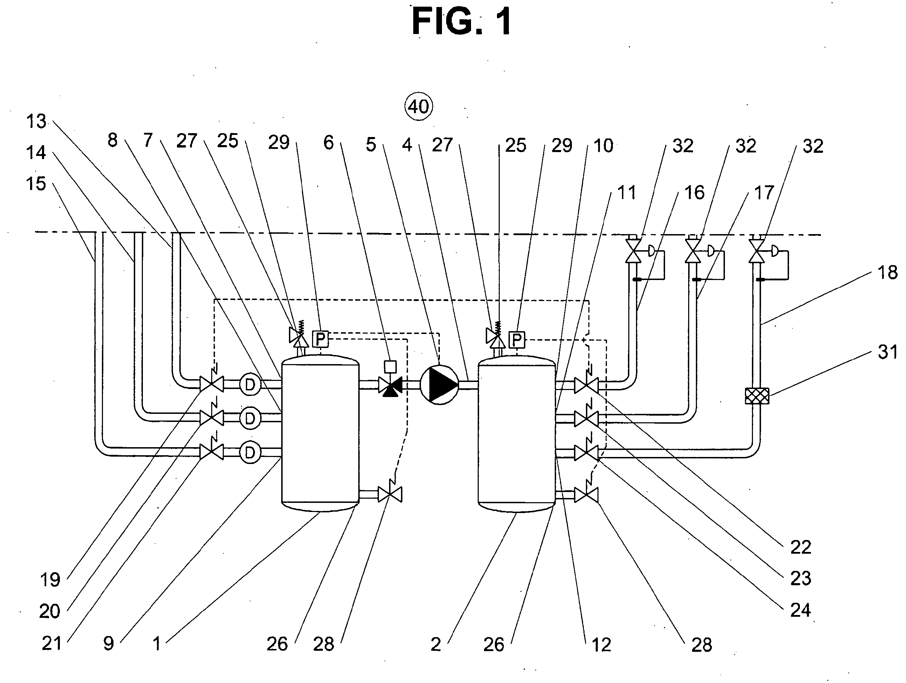

Dans l'exemple représenté à la figure 1, le dispositif comprend deux réservoirs 1, 2 disposés en série. Le réservoir 1 est relié à l'autre réservoir 2 par un circuit 4 de liaison équipé d'une pompe 5 à air qui aspire l'air dans le réservoir 1 pour le rejeter dans le réservoir 2. Le réservoir 1 est équipé de trois entrées 7, 8, 9 d'air raccordables chacune par un circuit 13, 14, 15 d'aspiration d'air obturable à l'enceinte à réguler, et le réservoir 2 de trois sorties 10, 11, 12 d'air raccordables chacune par un circuit 16, 17, 18 d'injection d'air obturable à l'enceinte à réguler.

Tous les organes d'obturation sont à commande en ouverture/fermeture indépendante d'un organe d'obturation à un autre.

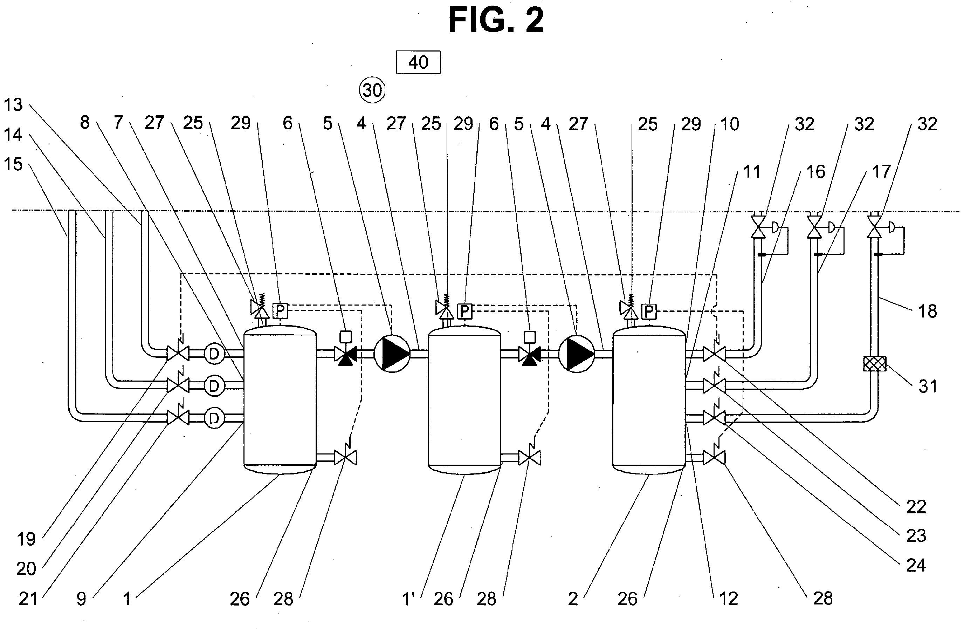

Dans l'exemple représenté à la figure 2, le dispositif comprend trois réservoirs 1, 1', 2 disposés en série. Le réservoir 1 le plus en amont de la série est relié à un réservoir 1' intermédiaire voisin par un circuit 4 de liaison équipé d'une pompe 5 à air apte à aspirer l'air dans le premier réservoir pour le rejeter dans le réservoir intermédiaire. Le réservoir intermédiaire est lui-même relié au dernier réservoir 2 de la série par un circuit 4 de liaison équipé d'une pompe 5 à air apte à aspirer l'air dans le réservoir intermédiaire pour le rejeter dans le dernier réservoir.

A nouveau, le premier et le dernier réservoirs de la série sont équipés, comme dans la figure 1, l'un, de trois entrées 7, 8, 9 d'air raccordables chacune par un circuit 13, 14, 15 d'aspiration d'air obturable à l'enceinte à réguler, l'autre, de trois sorties 10, 11, 12 d'air raccordables chacune par un circuit 16, 17, 18 d'injection d'air obturable à l'enceinte à réguler.

A chaque circuit d'admission d'air peut être assigné un rôle particulier. Ainsi, le circuit 13 d'admission et le circuit 16 d'injection peuvent être utilisés pour former un circuit de régénération apte à homogénéiser l'air entre les réservoirs et l'enceinte à réguler. Ce circuit est donc utilisé pour créer un début de fuite ou une reprise d'air permanente au niveau de l'enceinte par l'intermédiaire du réservoir 1 basse pression et une injection d'air permanente dans l'enceinte par l'intermédiaire du réservoir 2 haute pression.

L'organe 19 d'obturation du circuit 13 d'aspiration et l'organe 22 d'obturation du circuit 16 d'injection sont donc destinés à être ouverts de manière quasi permanente.

Les débits d'aspiration et d'injection peuvent être contrôlés pour rester sensiblement identiques. Ces débits peuvent être contrôlés par l'intermédiaire de moyens de mesure de pression disposés à l'intérieur de l'enceinte. Ils peuvent être également contrôlés par l'intermédiaire de moyens de mesure du débit disposés sur chacun desdits circuits.

Le circuit 14 d'aspiration et le circuit 17 d'injection peuvent être plus particulièrement destinés à permettre une variation importante de pression à l'intérieur de l'enceinte, tandis que le circuit 15 d'aspiration et le circuit 18 d'injection sont plus particulièrement destinés à permettre une variation faible de pression à l'intérieur de l'enceinte à réguler. Ces circuits peuvent éventuellement être dimensionnés de manière différente notamment au niveau des dimensions de conduit. Au moins certains circuits d'injection peuvent être équipés de moyens 31 de filtration et de détendeurs 32.

Les organes d'obturation sont à chaque fois formés ici d'électrovannes d'isolement de type tout ou rien.

Indépendamment du nombre de réservoirs, le circuit 4 de liaison entre deux réservoirs voisins de la série est un circuit obturable.

Dans les exemples représentés, l'organe 6 d'obturation du circuit 4 de liaison est une vanne trois voies commutable entre deux positions et apte à passer d'une position dans laquelle le réservoir immédiatement en aval dudit organe 6 d'obturation est apte à être alimenté en air à partir de l'air contenu dans le réservoir immédiatement en amont à une position dans laquelle le réservoir immédiatement en aval dudit organe 6 d'obturation est apte à être alimenté en air à partir d'air extérieur audit réservoir.

Par ailleurs, chaque réservoir 1, 1', 2 est équipé de deux sorties 25, 26 d'échappement d'air obturables débouchant à l'extérieur du dispositif, à l'air libre. L'organe 27 d'obturation de l'une des sorties d'échappement représentée en 25 aux figures est un organe d'obturation du type soupape sensible à la pression. Cet organe d'obturation chargé par ressort est apte à passer automatiquement de la position fermée à la position ouverte en fonction de la pression régnant à l'intérieur du réservoir.

L'organe 28 d'obturation de l'autre sortie 26 d'échappement d'air est à actionnement manuel ou commandé. Dans les exemples représentés, cet organe d'obturation est à nouveau formé par une électrovanne d'isolement de type tout ou rien à actionnement manuel.

Enfin chaque réservoir 1, 1', 2 est équipé de moyens 29 de mesure de la pression régnant à l'intérieur dudit réservoir et chaque pompe 5 à air est équipée de moyens de pilotage de ladite pompe en fonction des données fournies par lesdits moyens 29 de mesure.

Ces moyens 29 de mesure sont ici réalisés sous forme d'un pressostat équipant chaque réservoir.

Les moyens de pilotage de la pompe sont, quant à eux, configurés pour permettre le fonctionnement de la pompe 5 à air disposée sur le circuit de liaison entre le premier réservoir et le réservoir immédiatement en aval tant que la pression à l'intérieur du premier réservoir est supérieure à une pression prédéterminée, et de la pompe à air disposée sur le circuit de liaison entre le dernier réservoir et le réservoir immédiatement en aval tant que la pression à l'intérieur du dernier réservoir est inférieure à une pression prédéterminée, et ce dans le cas d'un dispositif comportant trois réservoirs.

Ces moyens de pilotage se présentent classiquement sous forme d'un système électronique et/ou informatique de commande de l'installation. Ce système peut comporter de manière classique des moyens de mémorisation des données, des moyens d'analyse des données et des moyens d'émission de signaux de commande des pompes en fonction du résultat de l'analyse effectuée par lesdits moyens d'analyse.

Ces moyens de pilotage peuvent comprendre une carte électronique munie d'un microprocesseur ou d'un microcontrôleur. Ainsi, lorsqu'il est précisé dans la description que ces moyens de pilotage sont configurés pour effectuer une opération donnée, cela signifie que le microprocesseur ou le microcontrôleur comprend des instructions informatiques permettant de réaliser ladite opération.

Dans l'exemple à deux réservoirs représenté, la pompe est pilotée pour maintenir une pression comprise par exemple entre - 0,5 et - 0,8 bar dans le réservoir 1 basse pression et une pression comprise entre 3,5 et 4 bar dans le réservoir 2, dit réservoir moyenne pression.

Dans le cas d'un dispositif à trois réservoirs, les pompes sont pilotées pour maintenir une pression comprise entre - 0,5 et - 0,8 bar dans le réservoir 1 basse pression, une pression comprise entre 3,5 et 4 bar dans le réservoir 1' moyenne pression, et une pression comprise entre 10 et 14 bar dans le réservoir haute pression.

Dans l'exemple du dispositif à trois réservoirs, le réservoir basse pression permet de réguler une augmentation de pression dans l'enceinte et d'avoir une homogénéité de l'air dans tout le dispositif. Il permet de lisser les variations de pression et d'avoir une régulation fine sur les augmentations de pression. Il sert de tampon lors des remontées importantes de température, ce qui évite d'avoir à utiliser une pompe à vide avec des débits très importants.

La pompe à air entre réservoirs basse pression et moyenne pression permet de garder une valeur de dépression constante dans le réservoir basse pression. Sa régulation se fait par rapport à deux seuils de pression dans le réservoir basse pression.

Cette pompe à air à la particularité de faire pompe à vide d'un côté et de faire compresseur de l'autre. Elle ramène un fluide compressé à 3,5 bar dans le réservoir 1' intermédiaire dit moyenne pression. Ce réservoir 1' intermédiaire moyenne pression sert de tampon et de stockage d'air.

La pompe à air entre réservoir 1' moyenne pression et réservoir 2 haute pression permet de faire monter la pression entre le réservoir moyenne pression et le réservoir haute pression. Elle comprime l'air jusqu'à 12 bar afin de pouvoir stocker un maximum d'air dans le réservoir haute pression.

Le réservoir 2 haute pression sert de stockage d'un volume d'air. Il a pour but de réguler une réduction de pression dans l'enceinte et d'avoir une homogénéité de la qualité de l'air dans tout le dispositif. Il permet de lisser les variations de pression et d'avoir une régulation fine sur les réductions de pression à l'intérieur de l'enceinte.

Le dispositif comporte encore des moyens 30 de mesure de la pression à l'intérieur de l'enceinte à réguler, tel qu'un capteur de pression.

Les moyens de commande en ouverture/fermeture des organes 19, 20, 21 et 22, 23, 24 d'obturation des circuits 13, 14, 15 d'aspiration et 16, 17, 18 d'injection sont aptes à émettre des signaux de commande desdits organes d'obturation en fonction des données fournies par les moyens 30 de mesure de la pression à l'intérieur de l'enceinte à réguler.

Ces moyens de commande peuvent être du même type que les moyens de pilotage décrits ci-dessus.

Généralement, le dispositif comporte deux modes de fonctionnement, l'un, dit mode de chargement en air des réservoirs 1, 1', 2 ou 1, 2, dans lequel la pompe 5 à air du ou d'au moins l'un des circuits 4 de liaison est raccordée en entrée à l'air extérieur, l'autre mode, dit de régulation, dans lequel la pompe 5 à air du ou de chacun des circuits 4 de liaison est raccordée en entrée au réservoir immédiatement en amont.

En pratique, le démarrage de l'installation et la régulation s'opèrent comme suit :
Au démarrage, les réservoirs 1' et 2 sont amenés à une pression comprise entre 3,5 et 4 bars pour le réservoir 1' et entre 10 et 14 bars pour le réservoir 2.

Cette étape de démarrage s'effectue à l'aide des pompes 5 qui sont raccordées en entrée à l'air extérieur. Ensuite, le réservoir 1 est amené à une pression entre -0,5 et -0,8 bar à l'aide de la pompe 5 disposée entre les réservoirs 1 et 1'.

Une fois les réservoirs à une pression moyenne telle que définie ci-dessus, les cycles de régulation et d'homogénéisation peuvent véritablement débuter.

On imagine dans un premier temps une augmentation de pression de la cellule climatique d'une valeur de ΔP.

Si cette valeur ΔP d'augmentation de la pression est inférieure à une valeur prédéterminée, la vanne 21 de liaison entre la cellule climatique et le premier réservoir 1 est ouverte pour permettre le passage de l'air de la cellule climatique au réservoir 1 jusqu'à ce que le ΔP soit égal à zéro.

Si le réservoir 1 augmente en pression jusqu'à une valeur supérieure à -0,5 bar, la pompe 5 disposée entre les réservoirs 1 et 1' est démarrée jusqu'à ce que la pression du réservoir 1 soit revenue à -0,8 bar.

Si la pression du réservoir 1' augmente jusqu'à une valeur supérieure à 4 bars, la pompe 5 disposée entre le réservoir 1' et le réservoir 2 démarre pour que la pression dans le réservoir 1' soit ramenée à 3,5 bars.

Le réservoir 2 sert, quant à lui, de réservoir tampon et de stockage du volume supplémentaire créé par la surpression.

Lorsque le ΔP est supérieur à une valeur prédéterminée, le fonctionnement est analogue à ce qui a été décrit ci-dessus mais la vanne qui est ouverte est la vanne 20 au lieu de la vanne 21 pour permettre une variation plus rapide de la pression à l'intérieur de la cellule climatique et une adaptation plus rapide du premier réservoir 1 en pression.

Dans le cas où, à l'inverse, la pression dans la cellule climatique diminue d'une pression ΔP, si le ΔP est inférieur à une valeur prédéterminée on ouvre dans un premier temps la vanne 24 de liaison du réservoir 2 et de la cellule climatique jusqu'à atteindre une valeur de pression dans la cellule climatique égale à la consigne demandée. Si le ΔP est supérieur à une valeur prédéterminée, on ouvre la vanne 23 au lieu d'ouvrir la vanne 24.

Le réservoir 2 doit donc être dimensionné et rempli en début d'expérience avec suffisamment de volume d'air afin de pouvoir redistribuer tout le volume nécessaire à l'expérience tout en tenant compte des fuites présentes au niveau de la cellule climatique.

Parallèlement à cette variation de pression à l'intérieur de l'enceinte, il est également possible de renouveler l'air à l'intérieur de cette enceinte.

A cet effet, la vanne 19 est ouverte en continu même lorsque la pression est équilibrée à l'intérieur de la cellule climatique, c'est-à-dire que le ΔP qui est égal à la pression de consigne à l'intérieur de la cellule moins la valeur de pression lue dans la cellule est égal à zéro.

La pompe 5 entre les réservoirs 1 et 1' se met en fonctionnement lorsque la pression du réservoir 1 augmente jusqu'à -0,5 bar pour s'arrêter à -0,8 bar qui correspond à la valeur de consigne de la pression.

La pompe 5 entre les réservoirs 1' et 2 se met en fonctionnement lorsque la pression du réservoir 1' atteint 4 bars pour ramener ladite pression à 3,5 bars. Le réservoir 2 sert de stockage de réserve en cas d'abaissement de pression.

L'organe d'obturation 22 est également ouvert afin d'assurer un appel d'air strictement identique au débit aspiré par l'organe d'obturation, formé par la vanne 19, afin de garantir une pression constante dans la cellule sans aucun à-coup.

Grâce à cette circulation en boucle sensiblement constante entre la cellule et les réservoirs, on assure une homogénéisation de la teneur en air de la cellule sans nuire à la possibilité de réguler la pression en air de ladite cellule en cas de dépression ou de surpression à l'intérieur de la cellule.

## Revendications

1. Dispositif pour l'homogénéisation et la régulation de l'atmosphère gazeuse d'une enceinte (40) étanche, telle qu'une cellule climatique,
**caractérisé en ce que** le dispositif comprend une pluralité de réservoirs (1, 1', 2) disposés en série, chaque réservoir étant relié à un réservoir voisin par un circuit (4) de liaison équipé d'une pompe (5) à air apte à aspirer l'air dans le réservoir, dit amont, de ladite paire de réservoirs voisins pour le rejeter dans le réservoir, dit aval, le réservoir le plus en amont de ladite série, dit premier réservoir (1), et le réservoir le plus en aval de la série, dit dernier réservoir (2), étant équipés respectivement l'un (1), d'au moins deux entrées (7, 8) d'air raccordables chacune par un circuit (13, 14), dit d'aspiration d'air, obturable, à l'enceinte à réguler, et l'autre (2), d'au moins deux sorties (10, 11) d'air raccordables chacune par un circuit (16, 17), dit d'injection d'air, obturable, à l'enceinte à réguler, les organes (19, 20) d'obturation des deux ou d'au moins deux des circuits (13, 14) d'aspiration étant du type à commande en ouverture/fermeture indépendante d'un organe d'obturation à un autre et les organes (22, 23) d'obturation des deux ou d'au moins deux des circuits (16, 17) d'injection étant du type à commande en ouverture/fermeture indépendante d'un organe d'obturation à un autre.

2. Dispositif selon la revendication 1,
**caractérisé en ce que** le dispositif comporte au moins deux modes de fonctionnement, l'un, dit mode de chargement en air des réservoirs (1', 2) dans lequel la pompe (5) à air du ou d'au moins l'un des circuits (4) de liaison est raccordée en entrée à l'air extérieur, l'autre, dit mode de régulation, dans lequel la pompe (5) à air du ou d'au moins l'un des circuits (4) de liaison est raccordée en entrée au réservoir immédiatement en amont.

3. Dispositif selon l'une des revendications précédentes,
**caractérisé en ce que** le réservoir le plus en amont de ladite série, dit premier réservoir (1), et le réservoir le plus en aval de la série, dit dernier réservoir (2), sont équipés respectivement l'un (1), d'au moins trois entrées (7, 8, 9) d'air raccordables chacune par un circuit (13, 14, 15) dit d'aspiration d'air obturable à l'enceinte à réguler et l'autre (2), d'au moins trois sorties (10, 11, 12) d'air raccordables chacune par un circuit (16, 17, 18) d'injection d'air obturable à l'enceinte à réguler.

4. Dispositif selon l'une des revendications précédentes,
**caractérisé en ce que** le ou au moins l'un des circuits (4) de liaison entre deux réservoirs de la série comporte un organe (6) d'obturation.

5. Dispositif selon la revendication 4,
**caractérisé en ce que** ledit organe (6) d'obturation du circuit (4) de liaison est une vanne trois voies commutable entre au moins deux positions, et est apte à passer d'une position dans laquelle le réservoir immédiatement en aval dudit organe (6) d'obturation est apte à être alimenté en air à partir de l'air contenu dans le réservoir immédiatement en amont, à une position dans laquelle le réservoir immédiatement en aval dudit organe (6) d'obturation est apte à être alimenté en air à partir d'air extérieur auxdits réservoirs.

6. Dispositif selon l'une des revendications précédentes,
**caractérisé en ce que** chaque réservoir (1, 1', 2) est équipé d'au moins une sortie (25, 26) d'échappement d'air obturable débouchant à l'extérieur du dispositif, à l'air libre.

7. Dispositif selon l'une des revendications précédentes,
**caractérisé en ce que** chaque réservoir (1, 1', 2) est équipé d'au moins deux sorties (25, 26) d'échappement d'air obturables débouchant à l'extérieur du dispositif, à l'air libre, l'organe (27) d'obturation de l'une (25) des sorties d'échappement étant un organe d'obturation de type soupape sensible à la pression et apte à passer automatiquement de la position fermée à la position ouverte en fonction de la pression régnant à l'intérieur du réservoir, l'organe (28) d'obturation de l'autre sortie (26) d'échappement étant à actionnement manuel ou commandé.

8. Dispositif selon l'une des revendications précédentes,
**caractérisé en ce que** chaque réservoir (1, 1', 2) est équipé de moyens (29) de mesure de la pression régnant à l'intérieur dudit réservoir et **en ce que** chaque pompe (5) à air est équipée de moyens de pilotage de ladite pompe en fonction des données fournies par lesdits moyens (29) de mesure de la pression.

9. Dispositif selon l'une des revendications 1 à 8,
**caractérisé en ce que** le dispositif comporte des moyens (30) de mesure de la pression à l'intérieur de l'enceinte (40) à réguler et **en ce que** les moyens de commande en ouverture/fermeture des organes d'obturation (19, 20, 21 ; 22, 23, 24) des circuits (13, 14, 15 ; 16, 17, 18) d'aspiration et d'injection sont aptes à émettre des signaux de commande desdits organes d'obturation en fonction des données fournies par les moyens (30) de mesure de la pression à l'intérieur de l'enceinte (40) à réguler.

10. Procécé d'homogénéisation et dé régulation de l'atmosphère gazeuse d'une enceinte (40) étanche, telle qu'une cellule climatique, à l'aide d'un dispositif conforme à l'une des revendications 1 à 9,
**caractérisé en ce que** les premier et dernier réservoirs (1, 2) étant respectivement, l'un (1), un réservoir basse pression, l'autre (2), un réservoir haute pression, ledit procédé comprend une étape de régénération de l'air de l'enceinte (40) à réguler par ouverture de l'un (13) des circuits d'aspiration et de l'un (16) des circuits d'injection d'air et une étape de régulation de l'atmosphère gazeuse de l'enceinte (40) par ouverture d'un circuit (14, 15) d'aspiration autre que celui (13) sollicité lors de l'étape de régénération et/ou par ouverture d'un circuit d'injection (17, 18) autre que celui (16) sollicité lors de l'étape de régénération en fonction de la pression régnant à l'intérieur de ladite enceinte (4) à réguler.

## Patentansprüche

1. Vorrichtung für das Vergleichmäßigen und die Regulierung der gasförmigen Atmosphäre eines dichten Behälters (40), wie eine Klimazelle,
**dadurch gekennzeichnet, dass** die Vorrichtung eine Vielzahl in Reihe angeordneter Vorratsbehälter (1, 1', 2) umfasst, wobei jeder Vorratsbehälter mit einem benachbarten Vorratsbehälter mittels eines Verbindungskreises (4) verbunden ist, der mit einer Luftpumpe (5) ausgestattet ist, die imstande ist, Luft in den vorgelagerten Vorratsbehälter des benachbarten Vorratsbehälterpaars anzusaugen, um sie in den nachgelagerten Vorratsbehälter auszustoßen, wobei der am meisten vorgelagerte erste Vorratsbehälter (1) der Reihe (1) und der am meisten nachgelagerte letzte Vorratsbehälter (2) der Reihe, jeweils einer (1), mit mindestens zwei Lufteinlässen (7, 8), die jeweils mittels eines verschließbaren Luftansaugkreises (13, 14) mit dem zu regulierenden Behälter verbindbar sind, und, der andere (2), mit mindestens zwei Luftauslässen (10, 11), die jeweils mittels eines verschließbaren Luftzufuhrkreises (16, 17) mit dem zu regulierenden Behälter verbindbar sind, ausgestattet sind, wobei die Verschlussorgane (19, 20) der zwei Kreise oder von mindestens zwei Ansaugkreisen (13, 14) der Bauart mit unabhängiger Steuerung des Öffnens/Schließens von einem Verschlussorgan zu einem anderen sind und die Verschlussorgane (22, 23) der zwei Kreise oder von mindestens zwei Zufuhrkreisen (16, 17) der Bauart mit unabhängiger Steuerung des Öffnens/Schließens von einem Verschlussorgan zu einem anderen sind.

2. Vorrichtung nach Anspruch 1,
**dadurch gekennzeichnet, dass** die Vorrichtung mindestens zwei Funktionsweisen aufweist, einen Modus der Befüllung der Vorratsbehälter (1', 2) mit Luft, bei dem die Luftpumpe (5) des oder von mindestens einem der Verbindungskreise (4) am Einlass mit der Außenluft verbunden ist, einen anderen Regelungsmodus, bei dem die Luftpumpe (5) des oder von mindestens einem der Verbindungskreise (4) am Einlass mit dem unmittelbar vorangehenden Vorratsbehälter verbunden ist.

3. Vorrichtung nach einem der vorangehenden Ansprüche,
**dadurch gekennzeichnet, dass** der am meisten vorgelagerte erste Vorratsbehälter (1) der Reihe (1) und der am meisten nachgelagerte letzte Vorratsbehälter (2) der Reihe, jeweils einer (1), mit mindestens drei Lufteinlässen (7, 8, 9), die jeweils mittels eines verschließbaren Luftansaugkreises (13, 14, 15) mit dem zu regulierenden Behälter verbindbar sind, und, der andere (2), mit mindestens drei Luftauslässen (10, 11, 12), die jeweils mittels eines verschließbaren Luftzufuhrkreises (16, 17, 18) mit dem zu regulierenden Behälter verbindbar sind, ausgestattet sind.

4. Vorrichtung nach einem der vorangehenden Ansprüche,
**dadurch gekennzeichnet, dass** der oder mindestens einer der Verbindungskreise (4) zwischen zwei Vorratsbehältern der Reihe ein Verschlussorgan (6) aufweist.

5. Vorrichtung nach Anspruch 4,
**dadurch gekennzeichnet, dass** das Verschlussorgan (6) des Verbindungskreises (4) ein Dreiwegeventil ist, das zwischen mindestens zwei Positionen schaltbar ist und imstande ist, von einer Position, in welcher der dem Verschlussorgan (6) unmittelbar nachgelagerte Vorratsbehälter imstande ist, mit Luft aus dem unmittelbar vorgelagerten Vorratsbehälter versorgt zu werden, in eine Position, in welcher der dem Verschlussorgan (6) unmittelbar nachgelagerte Vorratsbehälter imstande ist, aus der Außenluft außerhalb der Vorratsbehälter mit Luft versorgt zu werden, zu wechseln.

6. Vorrichtung nach einem der vorangehenden Ansprüche,
**dadurch gekennzeichnet, dass** jeder Vorratsbehälter (1, 1', 2) mit mindestens einem verschließbaren Luftablassauslass (25, 26) ausgestattet ist, der außerhalb der Vorrichtung in die freie Luft ausmündet.

7. Vorrichtung nach einem der vorangehenden Ansprüche,
**dadurch gekennzeichnet, dass** jeder Vorratsbehälter (1, 1', 2) mit mindestens zwei verschließbaren Luftablassauslässen (25, 26) ausgestattet ist, die außerhalb der Vorrichtung in die freie Luft ausmünden, wobei das Verschlussorgan (27) eines (25) der Ablassauslässe ein Verschlussorgan der Bauart Druckventil ist und imstande, in Abhängigkeit vom in Innern des Vorratsbehälters herrschenden Drucks automatisch aus der geschlossenen Position in die geöffnete Position zu wechseln, wobei das Verschlussorgan (28) des anderen (26) Ablassauslasses manuell oder gesteuert betätigt wird.

8. Vorrichtung nach einem der vorangehenden Ansprüche,
**dadurch gekennzeichnet, dass** jeder Vorratsbehälter (1, 1', 2) mit Messmitteln (29) des im Innern des Vorratsbehälters herrschenden Drucks ausgestattet ist und dass jede Luftpumpe (5) mit Steuermitteln der Pumpe in Abhängigkeit der von den Druckmessmitteln (29) bereitgestellten Daten ausgestattet ist.

9. Vorrichtung nach einem der Ansprüche 1 bis 8,
**dadurch gekennzeichnet, dass** die Vorrichtung mit Messmitteln (30) des Drucks im Innern des zu regulierenden Behälters (40) ausgestattet ist und dass die Steuermittel des Öffnens/Schließens der Verschlussorgane (19, 20, 21; 22, 23, 24) der Ansaug- und Zufuhrkreise (13, 14, 15; 16, 17, 18) imstande sind, Steuersignale der Verschlussorgane in Abhängigkeit von den von den Messmitteln (30) des Drucks im Innern des zu regulierenden Behälters (40) bereitgestellten Daten zu senden.

10. Verfahren für das Vergleichmäßigen und die Regulierung der gasförmigen Atmosphäre eines dichten Behälters (40), wie eine Klimazelle, mit Hilfe einer Vorrichtung nach einem der Ansprüche 1 bis 9,
**dadurch gekennzeichnet, dass** der erste und letzte Vorratsbehälter (1, 2) jeweils, einer (1), ein Niederdruck-Vorratsbehälter, der andere (2), ein Hochdruck-Vorratsbehälter sind, wobei das Verfahren einen Regenerationsschritt der Luft des zu regulierenden Behälters (40) durch Öffnen eines (13) der Ansaugkreise und eines (16) der Zufuhrkreise von Luft und einen Schritt der Regulierung der gasförmigen Atmosphäre des Behälters (40) durch Öffnen eines anderen Ansaugkreises (14, 15) als des während des Regenerationsschritts beanspruchten Kreises (13) und/oder durch Öffnen eines anderen Zufuhrkreises (17, 18) als des während des Regenrationsschritts beanspruchten Kreises (16) in Abhängigkeit von dem Druck, der in dem zu regulierenden Behälter (4) herrscht, umfasst.

## Claims

1. A device for homogenizing and adjusting the gaseous pressure of a sealed enclosure (40), such as a climate-controlled cell,
**characterized in that** the device comprises a plurality of reservoirs (1, 1', 2) arranged in series, each reservoir being connected to an adjacent reservoir by a connecting circuit (4) equipped with an air pump (5) able to suction air into the so-called upstream reservoir of said pair of adjacent reservoirs to discharge it into the so-called downstream reservoir, the furthest upstream reservoir of said series, called first reservoir (1), and the furthest downstream reservoir of the series, called last reservoir (2), respectively being equipped with one (1), with at least two air inlets (7, 8) each connectable by a circuit (13, 14), called air aspiration circuit, that can be closed off, to the enclosure to be adjusted, and the other (2), with at least two air outlets (10, 11) each connectable by a so-called air injection circuit (16, 17), which can be closed off, to the enclosure to be adjusted, the members (19, 20) for closing off the two or at least two of the suction circuits (13, 14) being of the type with independent opening/closing control from one closing off member to another and the members (22, 23) for closing off the two or at least two of the injection circuits (16, 17) being of the type with independent opening/closing control from one closing off member to another.

2. The device according to claim 1,
**characterized in that** the device includes at least two operating modes, one, called air charging mode of the reservoirs (1', 2), in which the air pump (5) or at least one of the connecting circuits (4) is connected at its inlet to the outside air, the other, said to be in adjusting mode, in which the air pump (5) or at least one of the connecting circuits (4) is connected at its inlet to the immediately upstream reservoir.

3. The device according to one of the preceding claims,
**characterized in that** the furthest upstream reservoir of said series, called first reservoir (1), and the furthest downstream reservoir of the series, called last reservoir (2), are respectively equipped with one (1), at least three (7, 8, 9) air inlets each connectable by a so-called inner aspiration circuit (13, 14, 15) that can be closed off to the enclosure to be adjusted and the other (2), with at least three air outlets (10, 11, 12) each connectable by an air injection circuit (16, 17, 18) that can be closed off to the enclosure to be adjusted.

4. The device according to one of the preceding claims,
**characterized in that** the or at least one of the connecting circuits (4) between two reservoirs of the series includes a closing off member (6).

5. The device according to claim 4,
**characterized in that** said member (6) for closing off the connecting circuit (4) is a three-way valve switchable between at least two positions, and is able to go from a position in which the reservoir immediately downstream from said closing off member (6) can be supplied with air from the air contained in the reservoir immediately upstream, to a position in which the reservoir immediately downstream from said closing off member (6) can be supplied with air from the air outside said reservoirs.

6. The device according to one of the preceding claims,
**characterized in that** each reservoir (1, 1', 2) is equipped with at least one air exhaust outlet (25, 26) that can be closed off emerging to the outside of the device, to the open air.

7. The device according to one of the preceding claims,
**characterized in that** each reservoir (1, 1', 2) is equipped with at least two air exhaust outlets (25, 26) that can be closed off emerging to the outside of the device, to the open air, the closing off member (27) of one (25) of the exhaust outlets being a closing off member of the pressure-sensitive gate type and able to go automatically from the closed position to the open position depending on the pressure prevailing inside the reservoir, the closing off member (28) of the other exhaust outlet (26) being actuated manually or in a controlled manner.

8. The device according to one of the preceding claims,
**characterized in that** each reservoir (1, 1', 2) is equipped with means (29) for measuring the pressure prevailing inside said reservoir and **in that** each air pump (5) is equipped with means for controlling said pump as a function of data provided by said pressure measuring means (29).

9. The device according to one of claims 1 to 8,
**characterized in that** the device includes means (30) for measuring the pressure inside the enclosure (40) to be adjusted and **in that** the means for controlling the opening/closing of the closing off members (19, 20, 21; 22, 23, 24) of the aspiration and injection circuits (13, 14, 15; 16, 17, 18) are able to emit control signals for said closing off members based on data provided by the means (30) for measuring the pressure inside the enclosure (40) to be adjusted.

10. A method for homogenizing and adjusting the gaseous atmosphere of a sealed enclosure (40), such as a climate-controlled cell, using a device according to one of claims 1 to 9,
**characterized in that** the first and last reservoirs (1, 2) respectively being a low-pressure reservoir (1) for one, and a high-pressure reservoir (2) for the other, said method comprises a step for regenerating air from the enclosure (40) to be adjusted by opening one (13) of the aspiration circuits and one (16) of the air injection circuits and a step for adjusting the gaseous atmosphere of the enclosure (40) by opening an aspiration circuit (14, 15) other than that (13) biased during the regenerating step and/or by opening an injection circuit (17, 18) other than that (16) biased during the regeneration step based on the pressure prevailing inside said enclosure (4) to be regulated.
